(19) Europäisches Patentamt
European Patent Office
Office européen des brevets

(11) Publication number: **0 126 849 A1**

(12) **EUROPEAN PATENT APPLICATION**

(21) Application number: 84101685.0

(22) Date of filing: 17.02.84

(51) Int. Cl.³: C 07 D 233/80
A 61 K 31/415

(30) Priority: 18.02.83 GB 8304521
16.08.83 GB 8322005

(43) Date of publication of application:
05.12.84 Bulletin 84/49

(84) Designated Contracting States:
AT BE CH DE FR GB IT LI LU NL SE

(71) Applicant: THE WELLCOME FOUNDATION LIMITED
183-193 Euston Road
London NW1 2BP(GB)

(72) Inventor: Caldwell, Albert Gordon
119 Gates Green Road
West Wickham Kent(GB)

(74) Representative: Sandmair, Kurt, Dr. Dr. et al,
Patentanwälte Dr. Berg Dipl.-Ing. Stapf Dipl.-Ing.
Schwabe Dr. Dr. Sandmair Postfach 86 02 45
Stuntzstrasse 16
D-8000 München 86(DE)

(54) **Pharmacologically active N-amino hydantoin derivatives, their synthesis and intermediates.**

(57) The hydantoins of formula (I) and their esters, amides, salts and solvates have prostaglandin mimetic and antagonistic properties rendering them useful in medicine. In formula (I)

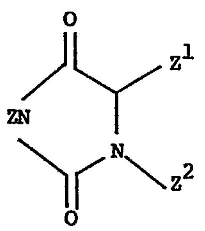

(I)

Z is hydrogen or alkyl;

$Z^1$ represents a group of formula $-CH_2-X-X^1-X^2$ wherein, X is selected from $-(CH_2)_2-$, and *cis* and *trans* $-CH=CH-$, $X^1$ is a covalent bond or a straight or branched alkylene chain having 1 to 6 carbon atoms optionally having one of its methylene groups replaced by oxa (−O−) or thia (−S−) provided that at least one carbon atom separates the oxa or thia group from a carboxyl or −CH=CH− group, and $X^2$ is a carboxyl group; and

$Z^2$ represents a group selected from $-NH-CH_2-R$ and $-N=CH-R$ wherein, R is a group selected from −CO−Y and

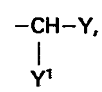

Y being a group selected from $C_{3-8}$ alkyl, $C_{3-8}$ alkenyl, phenyl−$C_{1-4}$ alkyl and phenyl (wherein the phenyl group in both cases is optionally substituted by one or more groups independently selected from alkyl, alkoxy, nitro, halo and trihalomethyl), cycloalkyl of 4 to 8 carbon atoms, and 5- or 6-membered heterocyclic radicals containing at least one heteroatom selected from oxygen, sulphur and nitrogen; and

$Y^1$ being a group selected from hydrogen, hydroxy, alkoxy and acyloxy.

EP 0 126 849 A1

# NEW HETEROCYCLIC COMPOUNDS, THEIR SYNTHESIS, COMPOSITIONS CONTAINING THEM AND THEIR USE IN MEDICINE

TITLE MODIFIED
see front page

This invention relates to novel hydantoin derivatives suitable for use as therapeutic agents, and to novel intermediates of use in the preparation thereof.

In UK patent specifications Nos.1,595,694; 1,595,695; 1,599,740 and 1,603,407, there are described certain hydantoin derivatives which have pharmacological properties related to those of natural prostaglandins, as demonstrated by their ability to mimic or antagonise the physiological effects of the natural prostaglandins in various biological preparations.

We have now discovered that a novel class of hydantoin derivatives, defined hereinbelow in formula (I), have particularly advantageous pharmacological properties such as are described below.

In formula (I)

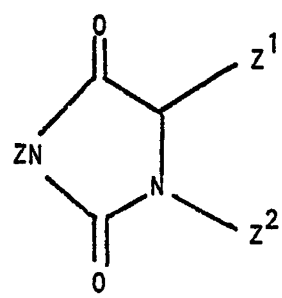

(I)

Z is hydrogen or alkyl;

$Z^1$ represents a group of formula $-CH_2-X-X^1-X^2$ wherein, X is selected from $-(CH_2)_2-$, and cis and trans -CH=CH-, $X^1$ is a covalent bond or a straight or branched alkylene chain having 1 to 6 carbon atoms optionally having one of its methylene groups replaced by oxa (-O-) or thia (-S-) provided that at least one carbon atom separates the oxa or thia group from a carboxyl or -CH=CH- group, and $X^2$ is a carboxyl group;

RMW/KMS/18.01.84

and $Z^2$ represents a group selected from $-NH-CH_2-R$ and $-N{=}CH-R$ wherein, R is a group selected from $-CO-Y$ and $-\underset{\substack{|\\ Y^1}}{CH}-Y$, Y being a group selected from $C_{3-8}$ alkyl, $C_{3-8}$ alkenyl, phenyl-$C_{1-4}$ alkyl and phenyl (wherein the phenyl group in both cases is optionally substituted by one or more groups independently selected from alkyl, alkoxy, nitro, halo and trihalomethyl), cycloalkyl of 4 to 8 carbon atoms, and 5- or 6- membered heterocyclic radicals containing at least one heteroatom selected from oxygen, sulphur and nitrogen; and

$Y^1$ being a group selected from hydrogen, hydroxy, alkoxy and acyloxy. The present invention also includes the esters, amides, salts and solvates (such as hydrates) corresponding to the compounds of formula (I). The present invention further includes bioprecursors or "pro-drugs" of the above-defined compounds according to the invention, namely compounds that are converted in vivo into compounds of formula (I) and their derivatives stated above.

Unless the context indicates to the contrary, any reference hereinafter to a hydantoin, is a reference to a hydantoin derivative of formula (I) and esters, amides, salts and solvates corresponding to such compounds. When intended for administration to the human or animal body (eg. as described further hereinbelow) such esters, amides, salts and solvates are those which are pharmacologically acceptable. Furthermore, in formula (I) and throughout this specification, unless specifically stated to the contrary, alkyl groups or the alkyl part of alkyl-containing moieties such as acyl, are straight or branched and contain from one to four carbon atoms. When Y is a 5- or 6- membered heterocyclic radical as defined above, the ring of the radical may be saturated or unsaturated, e.g. pyridyl, thienyl, tetrahydropyranyl or tetrahydrofuryl. When Y is an aralkyl group, this may be a phenylalkyl group, for example benzyl. The above-mentioned salts may be formed from those hydantoins of formula (I) wherein $X^2$ is carboxyl and Z is hydrogen or alkyl, or where $X^2$ is converted to the corresponding ester or amide and Z is hydrogen. Particularly valuable salts for medical purposes are those having a physiologically acceptable cation such as ammonium or that of an alkali metal e.g. sodium and potassium, an alkaline earth metal e.g. calcium or magnesium, or an organic base, particularly an amine such as tri(hydroxymethyl)aminomethane or ethanolamine. Salts having non-physiologically acceptable cations are included within the ambit of this invention as useful intermediates for the isolation or purification of the corresponding compounds of formula (I) or their physiologically acceptable salts. Except when there is clear indication to the contrary, formula (I) and other

formulae in the specification embrace all stereoisomers represented therein. In particular such formulae include geometrical isomers having the <u>syn</u> and <u>anti</u> configurations at the -N=CH- group, as well as diastereoisomers and individual enantiomers and racemic mixtures of these forms.

Preferred hydantoins of formula (I), by virtue of their advantageous pharmacological properties, include those wherein Z is hydrogen, $Z^1$ is $-CH_2-X-X^1-X^2$ wherein X is $-(CH_2)_2-$, $X^1$ is an alkylene chain of 2 to 4 carbon atoms and $X^2$ is carboxyl (or a corresponding alkyl ester) or a salt thereof;

and $Z^2$ is a group selected from $-NH-CH_2-R$ and -N=CH-R wherein R is a group of formula $-\underset{Y^1}{CH}-Y$ in which $Y^1$ is hydroxy and Y is phenyl, alkyl of 3 to 8 carbon atoms or cycloalkyl of 4 to 8, particularly 6 carbon atoms.

Within the scope of formula (I) is the sub-class of hydantoins wherein $Z^2$ represents $-NH-CH_2-R$ as defined, and esters, amides, salts and solvates thereof. A further sub-class comprises those hydantoins wherein $Z^2$ represents -N=CH-R as defined, and esters, amides, salts and solvates thereof.

Preferred hydantoins according to the invention include 5-(6-carboxyhexyl)-1-(2-cyclohexyl-2-hydroxyethylamino)hydantoin, 5-(6-carboxyhexyl)-1-(2-cyclohexyl-2-hydroxy-ethylideneamino)hydantoin, 5-(6-carboxyhexyl)-1-(2-hydroxy-2-phenylethylideneamino) hydantoin and their esters, amides, salts and solvates. The parent compounds may be provided in either of two isomeric forms, which for the first two of the aforementioned compounds are the diastereoisomer which is a viscous oil at $21^oC$ and the diastereoisomer which is a crystalline solid at $21^oC$, the former diastereoisomer being especially preferred.

The following hydantoins and their esters and salts are also preferred:-

5-(6-carboxyhexyl)-1-(2-cyclohexylethylideneamino)hydantoin
5-(6-carboxyhexyl)-1-(2-hydroxyheptylideneamino)hydantoin
5-(6-carboxyhexyl)-1-(2-hydroxy-2-phenylethylamino)hydantoin
5-(6-carboxyhexyl)-1-(2-cyclohexyl-2-hydroxyethylamino)-3-methylhydantoin

In common with other known hydantoins, those of formula (I) have pharmacological properties related to those of natural prostaglandins and can, for example, in their broad pharmacological profile, mimic or antagonise the biological effects of members of the prostaglandin (PG) 'A', 'B', 'C', 'D', 'E', 'F' and 'I' series. The

hydantoins according to the invention have been found to be advantageous in their pharmacological profile by virtue of their duration of action (which is considerably more prolonged than that of previously known hydantoin derivatives) and/or unexpected high potency. In this respect, 5-(6-carboxyhexyl)-1-(2-cyclohexyl-2-hydroxyethylideneamino)hydantoin and 5-(6-carboxyhexyl)-1-(2-hydroxy-2-phenylethylideneamino)hydantoin and their esters and salts according to the invention have been found to have an exceptionally prolonged duration of action. In general, compounds, of formula (I) wherein $Y^1$ is hydroxy have been found to exhibit the prolonged duration of action, and those wherein $Y^1$ is hydrogen have exhibited the unexpected high potency, although many of the compounds may exhibit both of these properties . The pharmacological profile, by which is meant the relative activities, mimetic or antagonistic, compared with the natural prostaglandins, will of course vary depending on the specific hydantoin under consideration. Therefore, having regard to the broad spectrum of activity of the natural prostaglandins, the potential utility of their antagonists and mimetics are diverse. Thus, the present invention may provide a hydantoin of formula (I) or a pharmacologically acceptable ester, amide, salt or solvate thereof for use in a method of prophylaxis or treatment of the human or animal body by therapy, especially where a prostaglandin antagonist or mimetic is indicated, eg for any of the clinical conditions described hereinafter.

A particular advantage of the hydantoins according to the present invention is that they exhibit a prolonged and potent anti-aggregatory effect on blood platelets. The hydantoins are thus useful whenever it is desired to inhibit platelet aggregation or to reduce the adhesive character of platelets, and may therefore be used for the treatment or prophylaxis of thrombo-embolic disorders in mammals, including man. The reference herein to "thrombo-embolic disorders" refers to disorders which are associated with undesired or increased platelet aggregation. In this role, it is believed that the hydantoins of formula (I) exhibit greater platelet binding than hydantoin derivatives previously known. The hydantoins are useful, for example, in the treatment and prevention of myocardial infarcts, thrombotic conditions, peripheral vascular disease, angina, as well as the promotion of patency of vascular grafts following surgery, and the treatment of complications of arteriosclerosis and conditions such as atherosclerosis, blood clotting defects due to lipemia, and other clinical conditions in which the underlying aetiology is associated with lipid imbalance or hyperlipidemia. A further use for such compounds is as additive to blood and other fluids which are used in artifical extra-corporeal circulation and perfusion of isolated body portions, to prevent platelet aggregation.

The hydantoins according to the present invention also cause relaxation of vascular smooth muscle in a similar way to members of the prostaglandin 'A' and 'E' series. Compounds relaxing vascular smooth muscle are capable of inducing vasodilation and therefore have antihypertensive properties and are useful in lowering the blood pressure in mammals, including man. Such compounds may be used alone or in combination with a β-adrenoceptor blocking agent or another antihypertensive substance for the treatment of all grades of hypertension including essential, malignant or secondary hypertension. It is advantageous for pharmaceutical agents intended for use in antihypertensive therapy, to have a prolonged duration of activity, and by virtue of their long-acting properties referred to above, the hydantoins of the invention are valuable in such a clinical role. The half-life of their vasodilatory effect which has been observed in experimental animals, has been found to be significantly greater than that produced by previously known prostaglandin mimetics of related structure. Furthermore, the observed decay profile has been found to be such that the total duration of the perceived vasodilatory effect is much greater relative to that produced by the known agents, than the half-life values would suggest. Hydantoins of formula (I) which inhibit pentagastrin-induced gastric acid secretion and reduce the formation of indomethacin-induced gastric lesions in rats are useful in reducing excessive gastric secretion, reducing and avoiding gastro-intestinal ulcer formation and accelerating the healing of such ulcers already present in the gastrointestinal tract whether such ulcers arise spontaneously or as a component of polyglandular adenoma syndromes.

In addition, the hydantoins of formula (I) may be used in the treatment of proliferative skin diseases such as are associated with excessive cell division in the epidermis or dermis which may be accompanied by incomplete cell differentiation.

Further applications of the hydantoins include inhibition of bronchoconstriction e.g. as induced by histamine or 5-hydroxytryptamine (5-HT). Hydantoins having this property may be used in the treatment or prophylaxis of bronchial asthma and bronchitis by alleviating the bronchoconstriction associated with such conditions. Hydantoins capable of antagonising histamine or 5-HT induced conditions may in general be used in the treatment or prophylaxis of diseases which result from anaphylactic reactions such as response to an allergen.

Hydantoins of formula (I) which mimic the uterine smooth muscle effects of $PGE_2$ and $PGF_{2\alpha}$ may be used as anti-fertility agents, in particular as abortifacients.

In some applications it is desirable that an effect or effects of a hydantoin other than its vasodilatory effect (for example an anti-platelet aggregatory effect) be potentiated relative to the vasodilatory effect. We have found that this may be achieved by formulating a composition comprising a hydantoin of formula (I) or a pharmacologically acceptable ester, amide, salt or solvate thereof, and an agent which is a cyclic AMP phosphodiesterase inhibitor (for convenience, hereinafter termed a phosphodiesterase inhibitor). Such a composition constitutes a further feature of the present invention.

Suitable phosphodiesterase inhibitors for use in potentiating the anti-aggregatory effects on the hydantoins include the following compounds as such, or as pharmacologically acceptable salts:-

(a) Xanthine derivatives such as:-
Theophyline (3,7-dihydro-1,3-dimethyl-1H-purine-2,6-dione), Caffeine (3, 7-dihydro-1, 3, 7-trimethyl-1H-purine-2, 6-dione): and Aminophylline (adduct of Theophylline and 1, 2-ethanediamine (2:1)).

(b) Isoquinoline derivatives, for example:-
Papaverine (1-[(3, 4-dimethoxyphenyl)methyl]-6, 7-dimethoxyisoquinoline);

(c) Derivatives of pyrimido [5, 4-d]-pyrimidine, for example:-
Dipyridamole (2, 2', 2", 2''' -[(4,8-dipiperidinopyrimido[5, 4-d]pyrimidin-2, 6-diyl)dinitrilo]-tetraethanol) and its salts;

(d) Derivatives of thieno [3, 2-d] pyrimidine, for example:-
N-[4-(4-morpholinyl)thieno[3, 2-d]pyrimidin-2-yl]-1, 2-ethanediamine.

(e) Derivatives of pyrazolo [3', 4':2, 3]pyrido-[4, 5-b]-[1, 5] benzodiazepin-6-(3H)one, for example:-
3-Ethyl-7, 12-dihydro-7, 12-dimethylpyrazolo-[4', 3':5, 6] pyrido [4, 3-b]benzo-diazepin-6-(3H)-one;

(f) Derivatives of 1H-or 2H-pyrazolo[3, 4-b]pyridine for example:-
4-(Butylamino)-1-ethyl-1H-pyrazolo[3, 4-b]pyridine-5-carboxylic acid ethyl ester; and 2-Methyl-6-phenyl-4-(1-piperidinyl)-2H-pyrazolo-[3, 4-b]pyridine;

(g) Derivatives of 5H-furo-[3, 4-e]pyrazolo-[3, 4-b]pyridine-5-one, for example:-
4-(Butylamino)-1-ethyl-1, 7-dihydro-7-hydroxy-5H-furo-[3, 4-e]pyrazolo[3, 4-b] pyridine-5-one; and

(h) Derivatives of 1-(2H)-naphthalenone, for example:-
2-(Dimethylamino)methyl-3, 4-dihydro-7-methoxy-1-(2H)-naphthalenone.

The amount of a hydantoin required to achieve the desired biological effect will of course depend on a number of factors, for example the specific compound chosen, the use for which it is intended, the mode of administration, and the recipient. In general, a daily dose may be expected to lie in the range of from 1 ng to 20 mg per kilogram bodyweight. For example, an intravenous dose may lie in the range of from 10 ng to 1 mg/kg which may conveniently be administered as an infusion of from 0.1 ng to 50 µg per kilogram per minute. Infusion fluids suitable for this purpose may contain from 0.01 ng to 100 µg, for example from 0.1 ng to 100 µg per millilitre. Unit doses may contain from 100 ng to 100 mg of a compound of formula (I), for example ampoules for injection may contain from 100 ng to 1 mg, and orally administrable unit dose formulations such as tablets or capsules may contain from 0.001 to 50, for example 0.02 to 20 mg.

More specifically, when a hydantoin is used to inhibit platelet aggregation, it is generally desirable to achieve a concentration in the appropriate liquid, whether it be the blood of a patient or a perfusion fluid, of from 10 ng to 10mg, for example from 100 ng to 1 mg, per litre.

The above-mentioned doses refer to the acids and esters, of formula (I); where a salt is used, the dose should be taken as referring to the corresponding anion.

For use in the treatment or prophylaxis of the conditions referred to above, while the hydantoin compounds may be used as the compound per se, they are preferably presented with an acceptable carrier therefor as a pharmaceutical formulation in accordance with the present invention. The carrier must of course be 'acceptable' in the sense of being compatible with the other ingredients of the formulation and not deleterious to the recipient thereof. The carrier may be a solid or a liquid, and is preferably formulated with a hydantoin compound as a unit-dose formulation, for example a tablet, which may contain from 0.05% to 95% by weight of the hydantoin compound. Other pharmacologically active substances may also be presented in formulations of the present invention as indicated above. The hydantoin compounds may be incorporated in the formulations either in the form of an acid, salt or ester thereof, and the formulations may be prepared by any of the well-known techniques of pharmacy consisting essentially of admixture of the components of the formulation.

The formulations include those suitable for oral, rectal, topical, buccal (e.g. sub-lingual), parenteral (e.g. subcutaneous, intramuscular, intradermal or

intravenous) administration, although the most suitable route in any given case will depend on the nature and severity of the condition being treated, and on the nature of the hydantoin compound.

Formulations suitable for oral administration may be presented in discrete units such as capsules, cachets, lozenges or tablets each containing a predetermined amount of hydantoin compound; as a powder or granules; as a solution or a suspension in an aqueous liquid or a non-aqueous liquid; as an oil-in-water emulsion; or as a water-in-oil liquid emulsion. Such formulations may be prepared by any of the methods of pharmacy but all methods include the step of bringing into association the hydantoin compound with the carrier which constitutes one or more accessory ingredients. In general they are prepared by uniformly and intimately admixing the hydantoin compound with liquid or finely divided solid carriers or both, and then, if necessary, shaping the product into the desired presentation. For example, a tablet may be prepared by compression or moulding a powder or granules of the hydantoin compound, optionally with one or more accessory ingredients. Compressed tablets may be prepared by compressing, in a suitable machine, the hydantoin compound in a free-flowing form such as a powder or granules optionally mixed with a binder, lubricant, inert diluent, surface active or dispersing agent(s). Moulded tablets may be made by moulding in a suitable machine, the powdered hydantoin compound moistened with an inert liquid diluent.

Formulations suitable for buccal (sub-lingual) administration include lozenges comprising a hydantoin compound in a flavoured basis, usually sucrose and acacia or tragacanth; and pastilles comprising a hydantoin compound in an inert basis such as gelatin and glycerin, or sucrose and acacia.

Formulations of the present invention suitable for parenteral administration conveniently comprise sterile aqueous preparations of a hydantoin compound, which preparations are preferably isotonic with the blood of the intended recipient. These preparations are preferably administered intravenously, although administration may also be effected by means of subcutaneous or intramuscular or intradermal injection. Such preparations may be conveniently prepared by admixing the hydantoin compound with water and rendering the product sterile and isotonic with the blood. Injectable compositions according to the invention will generally contain 0.1 to 5% w/w of active ingredient.

Formulations suitable for rectal administration are preferably presented as unit-dose

suppositories. These may be prepared by admixture of the hydantoin compound with one or more of the conventional solid carriers, for example cocoa butter, and shaping of the resulting mixture.

Formulations suitable for topical application to the skin preferably take the form of an ointment, cream, lotion, paste, gel, spray, aerosol or oil. Carriers which may be used include vaseline, lanoline, polyethylene glycols, alcohols and combinations thereof. The active ingredient is generally present in a concentration of from 0.1 to 15% w/w of the composition, for example from about 0.5 to about 2%.

The hydantoins may be prepared in any conventional manner and in accordance with the present invention, may for example be prepared by any method hereinafter described. Thus, according to the present invention we provide a process for the preparation of hydantoins of formula (I) and their above-defined salts which comprises:-

(a) for the preparation of hydantoins where $Z^2$ represents -N=CH-R, wherein R is as defined above, reacting of a compound of formula (II)

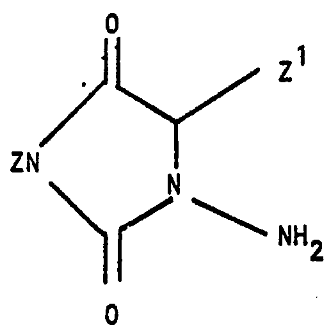

(II)

(wherein Z and $Z^1$ are as defined above) with a compound of formula (III)

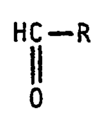

(III)

RMW/KMS/18.01.84

(wherein R is as defined above);

(b) for the preparation of hydantoins where $Z^2$ represents -N=CH-R, wherein R is as defined above, reacting an appropriate compound of formula (IV)

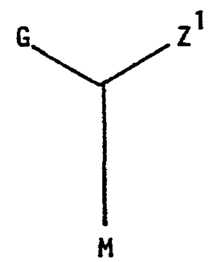

(IV)

(wherein $Z^1$ is as defined above, G is carboxy or a derivative thereof such as an amide derivative e.g. carbamoyl, or an ester thereof, particularly an alkyl ester or G may be cyano and M is a leaving group such as halo, preferably bromo) with a compound of formula (V)

ZHNCONHN=CHR (V)

(wherein R and Z are as defined above);

(c) for the preparation of hydantoins where $Z^2$ represents-N=CH-R, wherein R is as defined above, cyclising a compound of formula (VI)

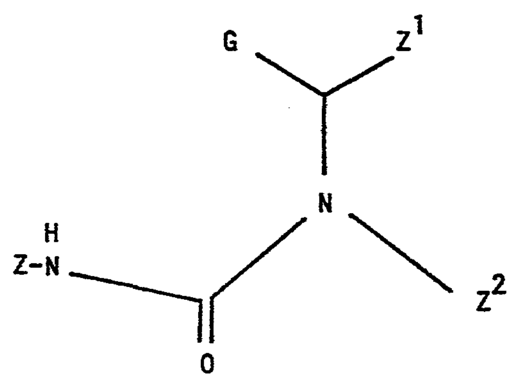

(VI)

wherein G, Z, $Z^1$ and $Z^2$ are as defined above.

(d) for the preparation of hydantoins where $Z^2$ represents -N=CH-R, wherein R is as defined above, and Z is as defined above, treating a compound of formula (VII)

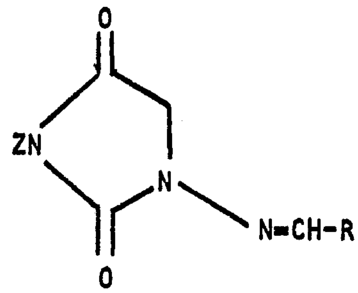

(VII)

(wherein R is as defined above) with methylmagnesium carbonate followed by reaction with a compound of formula (VIII)

$M-Z^1$ (VIII)

(wherein M and $Z^1$ are as defined above) and subsequently, if desired, effecting one or more of the following optional conversions in any desired order:-

i) where a hydantoin of formula (I) is formed and is an ester, converting said compound into a corresponding acid, or to a salt, solvate or amide thereof;

ii) where a hydantoin of formula (I) is formed and is an acid, converting said hydantoin into a corresponding ester, or to a salt, solvate or amide thereof;

iii) where a hydantoin of formula (I) is formed wherein Z is hydrogen, converting said hydantoin into a corresponding hydantoin wherein Z is alkyl;

iv) where a hydantoin of formula (I) is formed wherein R is $-\underset{Y^1}{CH}-Y$, wherein $Y^1$ is an acyloxy group and Y is as defined above, converting said hydantoin into a corresponding hydantoin of formula (I) wherein $Y^1$ is a hydroxy group;

v) where a hydantoin of formula (I) is formed wherein R is a group -CO-Y, converting the said hydantoin into a corresponding hydantoin of formula (I) wherein R is a group -CH(OH)-Y; or

(vi) converting the hydantoin of formula (I) to the corresponding hydantoin where $Z^2$ represents $-NH-CH_2-R$, wherein R is as defined above.

The reaction in process (a) may be conveniently performed in an appropriate solvent, e.g. methanol.

The reaction in process (b) may be carried-out by heating in the presence of a suitable base such as an alkali-metal alkoxide, e.g. sodium ethoxide and optionally in the presence of a suitable solvent such as ethanol;

In process (c) the carboxyl derivative may, for example, be an amide or ester, in particular, an alkyl ester. The cyclisation may be performed under acidic conditions or by heating alone. The reaction may be effected in the absence of a solvent, but if desired an inert solvent may be used, for example, a hydrocarbon such as petrol. Alternatively, where G is alkoxycarbonyl, cyclisation may be effected in the presence of a suitable base, for example an alkoxide such as sodium ethoxide.

In the process (d) the compound of formula (VI) may be in a suitable solvent such as dimethylformamide.

If, in the above process, it is desired to separately isolate one or both diastereoisomers of a compound of formula (I), where an appropriate compound exists in mixed isomeric form, separation of the isomers may conveniently be performed in conventional manner, e.g. by h.p.l.c.

Compounds of formula (II) may be prepared by hydrogenation of a compound of formula (IX)

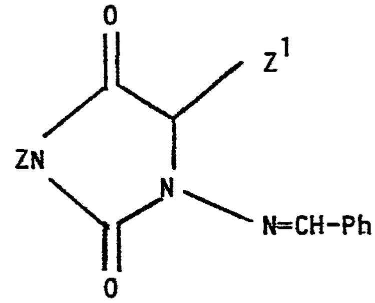

(IX)

(wherein Z and $Z^1$ are as defined above, and Ph represents phenyl) under suitable conditions. Conveniently, such hydrogenation may be performed at elevated pressure in the presence of a palladium/charcoal catalyst. If desired, the reaction may be carried out with heating.

A compound of formula (II) may also be prepared by reaction of a compound of formula (X)

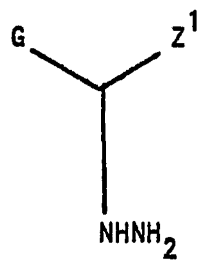

(X)

(wherein G is as defined above) with cyanic acid or an alkyl isocyanate, depending respectively, on whether Z is hydrogen or alkyl.

When cyanic acid is used, the cyanic acid is conveniently produced in situ by the use of an alkali metal cyanate, e.g. potassium cyanate, and an acid which may be present as an acid addition salt of the compound of formula (X) or a free acid of formula (X) wherein either or both of R and $X^2$ is hydrogen. Alternatively an equivalent amount of mineral acid or an organic acid may be added to the reaction medium. The reaction may proceed in the absence of a solvent but desirably an inert solvent is used which is preferably polar such as water or a mixture of water with acetone, dimethylformamide, dimethylsulphoxide or a lower alkanol such as ethanol or it may be a hydrocarbon, an ether or halogenated hydrocarbon such as chloroform. Where desired, for example if no solvent is used, the reaction may be promoted by heating the reactants.

Similar reaction conditions may be used when an alkyl iso-cyanate is used except that it is unnecessary to provide an equivalent amount of acid, as an acid addition salt or otherwise, in the reactants.

Instead of using a cyanate or isocyanate, a compound of formula (X) may be reacted with urea, nitrourea of an N-alkylurea as appropriate. A solvent is not essential but if desired an inert solvent such as one mentioned above may be used, and the reaction is preferably effected at an elevated temperature, for example from $100^{\circ}$ to $125^{\circ}C$ but temperatures upto $150^{\circ}C$ may be employed.

Compounds of formula (IX) may be prepared by reaction of a compound of formula (IV) (as previously defined) with a compound of formula (XI)

ZHNCONHN=CHPh (XI)

wherein Z is as defined above and Ph represents phenyl.

The compounds of formulae (V) and (XI) may be prepared, for example, by reaction of the appropriate aldehyde with semicarbazide, if necessary under mildly acid conditions.

A compound of formula (IV) may conveniently be prepared in a manner analogous to that described by Schwenk and Papa in J.Amer.Chem.Soc., 1948, 70, 3626.

A compound of formula (X) may be prepared by reaction of a compound of formula (IV) as previously defined, with an excess of hydrazine hydrate under appropriate conditions, in a suitable solvent such as ethanol.

The compounds of formula (III) may be prepared in a manner analogous to that for example described by Tiffany *et al* in J.Amer. Chem. Soc. 1957, 79, 1682, or Royals and Robinson in J.Amer.Chem.Soc., 1956, 78, 4161.

A compound of formula (VI) may be prepared by reaction of a compound of formula (XII)

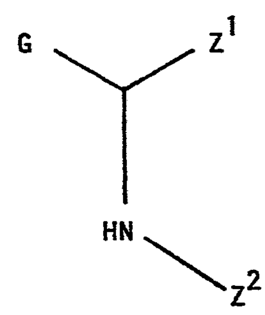

(XII)

(wherein G, $Z^1$ and $Z^2$ are as defined above) with cyanic acid or an alkylisocyanate, depending respectively, on whether Z is hydrogen or alkyl. The reaction may be performed in a manner, and under conditions, analogous to those previously described for the reaction of a compound of formula (X) to form a compound of formula (II). In this synthesis, a compound of formula (VI) need not be isolated from the reaction mixture and may be converted directly to a compound of formula (I) under the described reaction conditions.

A compound of formula (XII) may be conveniently prepared by reaction of a compound of formula (X) as previously defined, with a compound of formula (III) as previously defined, under reaction conditions analogous to those described for process (a).

A compound of formula (VII) may be prepared by reaction of a compound (XIII)

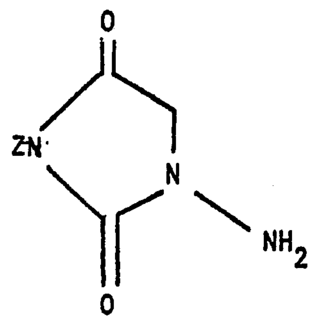

(XIII)

with a compound of formula (III) as previously defined, in conventional manner.

A compound of formula (XIII) may be prepared according to the method described by Jack in J.Pharm.Pharmacol, 1959, 108T and where appropriate, alkylating the ring at the 3- position in conventional manner.

A compound of formula (VIII) may for example be prepared in a manner analogous to that described by Bowman in J.Chem.Soc., 1950 p.174.

In optional conversion (i) above, the reaction is advantageously effected by hydrolysis, e.g. under basic conditions, for example in the presence of sodium hydroxide. Similar hydrolysis may be employed in optional conversion (iii) where a $Y^1$ acycloxy group is converted into a $Y^1$ hydroxy group.

In optional conversion (ii), the reaction is advantageously effected by treatment with the appropriate alcohol (e.g. ethanol) in the presence of a suitable acid such as sulphuric.

In either of conversions (i) or (ii) the appropriate carboxylate salt may be formed by treatment with an appropriate inorganic or organic base in an

appropriate solvent such as water, followed by isolation, e.g. by methanol precipitation, evaporation or freeze-drying.

In conversion (iii), the reaction is advantageously effected by treatment with a suitable alkylating agent such as an alkyl halide (eg. the iodide) in the presence of a suitable base such as sodium hydroxide.

In conversion (iv), deacylation may be performed in conventional manner, e.g. by treatment with sodium hydroxide.

Conversion (v) may be effected by conventional reduction using an agent such as sodium borohydride under basic conditions.

In optional conversion (vi), the reaction may be effected using any appropriate reducing agent such as the borohydride or cyanoborohydride of an alkali metal e.g. sodium or lithium. The reaction proceeds under suitable conditions which may, in the case of cyanoborohydrides, require the presence of an acid, e.g. acetic acid.

In general, the reactions and conversions specified above may be effected in conventional manner using techniques known for the preparation of analogous compounds.

The above defined intermediate compounds of formulae (II) and (IX) are new and represent further features of the present invention.

In all of the foregoing chemical procedures it is of course evident that the choice of reactant will be dictated in part by the functional groups present in the substrate, and where necessary reactants having an appropriate selectivity of action must be used.

It will be appreciated from the foregoing that what we may claim can comprise, for example:-

(a) Any novel hydantoin of formula (I) as hereinabove defined.

(b) A method for the preparation of the novel hydantoins of formula (I) as hereinabove described.

(c) A pharmaceutical formulation comprising a hydantoin of formula (I) in association with a pharmaceutically acceptable carrier therefor, and methods for the preparation of such formulations.

(d) A method for lowering blood pressure in a mammal such as man which comprises administration to the mammal of an effective hypotensive, non-toxic amount of a hydantoin of formula (I).

(e) A method for the treatment or prophylaxis of thrombosis in a mammal or mammalian tissue such as human, which comprises administration of a non-toxic, effective anti-thrombotic amount of a hydantoin of formula (I).

(f) A method for inducing vasodilation in a mammal, such as man, comprising administration to said mammal of a non-toxic effective vasodilatory amount of a hydantoin of formula (I).

(g) A method for the treatment or prophylaxis of gastric lesions in a mammal such as man comprising administration to said mammal of a non-toxic effective prophylactic or therapeutic amount of a hydantoin of formula (I).

(h) A method for inducing bronchodilation in a mammal, such as man, comprising administration to said mammal of a non-toxic, effective bronchodilatory amount of a hydantoin of formula (I).

(i) A method for the treatment or prophylaxis of an allergic condition in a mammal, such as man, comprising administration to said mammal of a non-toxic effective prophylactic or therapeutic amount of a hydantoin of formula (I).

(j) A method of inducing abortion of a foetus in a mammal, eg human, comprising administration to said mammal of a non-toxic effective abortifacient amount of a hydantoin of formula (I).

(k) A method of inducing infertility in a mammal, eg human comprising administration to said mammal of a non-toxic effective contraceptive amount of a hydantoin of formula (I).

(l) A method of treating a proliferative skin disease in a mammal which comprises bringing an effective therapeutic amount of a hydantoin of formula (I) into the proximity of the skin lesion.

(m) A method of treating angina in a mammal comprising the administration to said mammal of a non-toxic therapeutic amount of a hydantoin of formula (I).

(n) Any diastereoisomer of a hydantoin of formula (I) or any mixture thereof.

(o) A hydantoin of formula (I) as hereinabove defined and pharmaceutically and pharmacologically acceptable salts thereof, for use in lowering blood pressure in a mammal such as man.

(p) A hydantoin of formula (I) as hereinabove defined and pharmaceutically and pharmacologically acceptable salts thereof, for use in the treatment or prophylaxis of thrombosis in a mammal or mammalian tissue, such as human.

(q) A hydantoin of formula (I) as hereinabove defined and pharmaceutically and pharmacologically acceptable salts thereof, for use in inducing vasodilation in a mammal, such as man.

(r) A hydantoin of formula (I) as hereinabove defined and pharmaceutically and pharmacologically acceptable salts thereof, for use in the treatment or prophylaxis of gastric lesions in a mammal such as man.

(s) A hydantoin of formula (I) as hereinabove defined and pharmaceutically and pharmacologically acceptable salts thereof, for use in inducing bronchodilation in a mammal such as man.

(t) A hydantoin of formula (I) as hereinabove defined and pharmaceutically and pharmacologically acceptable salts thereof, for use in the treatment or prophylaxis of an allergic condition in a mammal such as man.

(u) A hydantoin of formula (I) as hereinabove defined and pharmaceutically and pharmacologically acceptable salts thereof, for use in inducing abortion of a foetus in a mammal such as human.

(v) A hydantoin of formula (I) as hereinabove defined and pharmaceutically and pharmacologically acceptable salts thereof, for use in inducing infertility in a mammal such as human.

(w) A hydantoin of formula (I) as hereinabove defined and pharmaceutically and pharmacologically acceptable salts thereof, for use in treating a proliferative skin disease in a mammal such as man.

(x) A hydantoin of formula (I) as hereinabove defined and pharmaceutically and pharmacologically acceptable salts thereof, for use in treating angina in a mammal such as man.

(y) A composition comprising a hydantoin of formula (I) as hereinbefore defined or a pharmaceutically or pharmacologically acceptable salt thereof, and a cyclic AMP phosphodiesterase inhibitor.

(z) Any novel feature herein described.

The present invention will now be illustrated by way of the following examples.

Example 1

Preparation of 5-(6-carboxyhexyl)-1-(2-cyclohexyl-2-hydroxyethylideneamino) hydantoin

(a) 1-Benzylideneamino-5-(6-ethoxycarbonylhexyl)hydantoin

A solution of sodium ethoxide was prepared by dissolving sodium (4.6 g) in ethanol (100 ml). A suspension of benzaldehyde semicarbazone (16.3 g) in the sodium ethoxide solution (50 ml) and ethanol (20 ml) was refluxed for 15 minutes. Diethyl 2-bromononanedioate (16 g) was added and the mixture was refluxed for 30 minutes. Sodium ethoxide solution (25 ml) was added and the mixture was refluxed for 5 minutes before the addition of the bromodiester (8 g) followed by 30 minutes' refluxing. The remaining sodium ethoxide solution (25 ml) and bromodiester (8 g)

were then added and the mixture was refluxed for 1 hour. Most of the ethanol was evaporated *in vacuo* and the residue was shaken with dilute hydrochloric acid and ether. Unchanged benzaldehyde semicarbazone (5.4 g) was filtered off, and the ether solution was washed with water, dried ($MgSO_4$) and evaporated. The semi-solid residue was treated with a small volume of ether, to give 1-benzylideneamino-5-(6-ethoxycarbonylhexyl)hydantoin (13.0 g), crystallising from cyclohexane in colourless needles, m.p. 92-94°C.

(b) 1-Amino-5-(6-ethoxycarbonylhexyl)hydantoin

The above benzylideneamino- compound (5.8 g) in solution in ethanol (100 ml) was hydrogenated at 10 atmospheres pressure and 50°C in the presence of 10% palladium-on-charcoal catalyst for 1 hour. The catalyst was filtered off and the solvent was evaporated to leave the amino-compound (4.3 g) which crystallised from ethyl acetate-light petroleum (b.p. 60-80°C), in colourless needles, m.p. 80-82°C.

(c) 5-(6-Carboxyhexyl)-1-(2-cyclohexyl-2-hydroxyethylideneamino)hydantoin.

Method (i)

A solution of 1-amino-5-(6-ethoxycarbonylhexyl)hydantoin (270 mg) and cyclohexylglyoxal hemihydrate (Tiffany *et al* J.Amer.Chem.Soc. 1957, 79, 1682) (140 mg) in methanol (1.5 ml) was refluxed for 1 hour. Water was added and the precipitated solid (330 mg) was recrystallised from aqueous methanol to give colourless plates, m.p. 88-90°C, of 5-(6-ethoxycarbonylhexyl)-1-(cyclohexylcarbonylmethyleneamino)hydantoin (Compound No. 1).

This ester (790 mg) was hydrolysed in 0.5N-sodium hydroxide solution (4ml) at room temperature for 1 hour. The solution was washed with ether, acidified with N-hydrochloric acid and the precipitated gum was extracted into ether. The washed and dried ether solution was evaporated to leave a colourless solid (730mg), which was recrystallised from aqueous methanol to give colourless plates, m.p. 150-152°C, of 5-(6-carboxyhexyl)-1-(cyclohexylcarbonylmethyleneamino)hydantoin (Compound No. 2).

The keto-acid (1 g), in solution in 0.5N-sodium hydroxide solution (13.7 ml), was treated with sodium borohydride (52 mg), added in portions, and the solution was stirred for 3 hours at room temperature. After acidification with 2N-hydrochloric acid, the precipitated oil was extracted into chloroform. The washed and dried extract was evaporated to leave a colourless viscous oil (700mg), which when

treated with ether gave a solid. Recrystallisation from aqueous methanol gave one of the pure diastereoisomers of 5-(6-carboxyhexyl)- 1-(2-cyclohexyl-2-hydroxy-ethylideneamino)hydantoin (Compound No. 3) as colourless plates, m.p. 84-86$^{o}$C NMR ($CDCl_3$): δ 2.34 (2H,t,$C\underline{H}_2CO_2H$), 4.16 (1H,t,$C\underline{H}OH$), 4.37(1H,t,COCHN), 8.37(1H,d,N=CH)

Method (ii)

A solution of 1-amino-5-(6-ethoxycarbonylhexyl)hydantoin (3.0 g) and 2-ace-toxy-2-cyclohexylacetaldehyde (Ross *et al*, J.Med.Chem. 1979, 22, 412) (2.24 g) in methanol (20 ml) was refluxed for 1 hour. The methanol was evaporated, the residue was dissolved in ether, and the ether solution was washed with N-hydrochloric acid, then water, and dried. The oil remaining after evaporation of the ether solution was purified by chromatography on a column of silica in a mixture of chloroform and methanol (40:1) to give a colourless oil (4.9 g) showing two spots Rf 0.46 and 0.50 on t.l.c. (silica; chloroform-methanol-acetic acid (95:4:1)). Separation of a portion (1.5 g) by h.p.l.c. (silica; dichloromethane- methanol-acetic acid (98.5:1.0:0.5)) gave the individual diastereoisomers of 1-(2-acetoxy-2-cyclohexylethylideneamino)-5-(6-ethoxycarbonylhexyl)hydantoin as colourless viscous oils, Rf 0.50 (625 mg) (Compound No.4) and Rf 0.46 (850 mg)(Compound No. 5).

The pure isomer, Rf 0.50 (100 mg) was dissolved in 0.5N-sodium hydroxide solution (2 ml). After 1 hour at room temperature the solution was washed with ether, then acidified with N-hydrochloric acid and the precipitated oil was extracted into chloroform. The washed and dried chloroform solution was evaporated to leave one of the almost pure diastereoisomers of 5-(6-carboxyhe-xyl)-1-(2-cyclohexyl-2-hydroxyethylideneamino)hydantoin(Compound No. 6) as a colourless viscous oil. NMR ($CDCl_3$):δ2.32(2H,t,$C\underline{H}_2CO_2H$), 4.17(1H,t,$C\underline{H}OH$), 4.39(1H,t, COCHN), 8.23 (1H,d,N=CH).

Similarly, the diastereoisomer, Rf 0.46, of the acetoxy-ester was hydrolysed to give the second diastereoisomer of 5-(6-carboxyhexyl)-1-(2-cyclohexyl-2-hydroxy-ethylideneamino) hydantoin, m.p. 84-86$^{o}$C, identical with that obtained in method (i) above (Compound No. 3).

Example 2

Preparation of 5-(6-Carboxyhexyl)-1-(2-cyclohexylethylideneamino)hydantoin

A solution of 1-amino-5-(6-ethoxycarbonylhexyl)hydantoin (271 mg) and cyclohexylacetaldehyde (126 mg) in methanol (4 ml) was refluxed for 2 hours. The solvent was evaporated and the residual solid was recrystallised from methanol to give a colourless needles, m.p. 95-97$^{o}$C, of 1-(2-cyclohexylethylidene-amino)-5-(6-ethoxycarbonylhexyl)hydantoin (Compound No.7).

This ester (380 mg) in solution in aqueous sodium hydroxide (11 ml of 0.2 N) was left at room temperature for 2 hours. The solution was washed with ether, acidified with 2 N-hydrochloric acid and the precipitated oil was extracted into ether. The ether solution was washed with water, dried over magnesium sulphate and evaporated to leave 5-(6-carboxyhexyl)-1-(2-cyclohexylethylideneamino) hydantoin, (Compound No.8) which crystallised from a mixture of di-isopropyl ether and light petroleum (b.p. 60-80$^{o}$C) as colourless needles, m.p. 90-92$^{o}$C.

Example 3

Preparation of 5-(6-Carboxyhexyl)-1-(2-hydroxy-2-phenylethylideneamino)hydantoin

Method (i)

A solution of 1-amino-5-(6-ethoxycarbonylhexyl)hydantoin (1.95 g) and α-acetoxyphenylacetaldehyde (Riehl and Fougerousse, Bull.Soc.Chim., 1968, 4083) (1.3 g) in methanol (15 ml) was refluxed for 2 hours. The solvent was evaporated and the pale yellow oil remaining was purified by chromatography on a column of silica with a mixture of chloroform and methanol (50:1) as eluant to give 5-(6-ethoxycarbonylhexyl)-1-(2-acetoxy-2-phenylethylideneamino)hydantoin as an oily mixture of diastereoisomers showing spots at Rf 0.28 (Compound No.9) and 0.31 (Compound No.10) on thin-layer chromatography (silica; chloroform-methanol (50:1)). By use of high performance liquid chromatography (silica; dichloromethane-methanol (100:1)) the diastereoisomers were separated : less polar (Compound No. 10), n.m.r. ($CDCl_3$) : δ 2.23 (5H, m, $CH_2CO_2$ and $COCH_3$), 4.11 (2H, qu, $CO_2C\underline{H}_2CH_3$)$_1$, 4.38 (1H,t, N-CH), 6.41 (1H,d, CH-O) 7.38 (5H, s,Ph), 8.44 (1H,d, N=CH); more polar (Compound No.9) 2.21 (5H, m, $CH_2CO_2$ and $COCH_3$), 4.11 (2H, qu, $CO_2C\underline{H}_2CH_3$), 4.43 (1H,t, N-CH), 6.40 (1H,d, CH-O), 7.38 (5H, s, Ph), 8.35 (1H,d, N=CH).

RMW/KMS/16.01.84

The pure less polar acetoxy-ester (170 mg) was hydrolysed in aqueous sodium hydroxide (6.5 ml of 0.2N) at room temperature for 1 hour. The solution was washed with ether and acidified with 2N-hydrochloric acid. The precipitate was extracted into ethyl acetate, the extract was washed with water, dried, (magnesium sulphate) and evaporated to leave a colourless solid. Recrystallisation from ethyl acetate - light petroleum (b.p. 60-80°C) gave needles, m.p. 112-114°C, of one diastereoisomer of 5-(6-carboxyhexyl)-1-(2-hydroxy-2-phenylethylideneamino)hydantoin (Compound No.11).

Similarly, the more polar acetoxy-ester was hydrolysed to give the second diastereoisomer of 5-(6-carboxyhexyl)-1-(2-hydroxy-2-phenylethylideneamino) hydantoin (Compound No 12), which crystallised from ethyl acetate - light petroleum (b.p.60-80°C) as small colourless needles of the monohydrate m.p. 70-73°C.

Method (ii)

Under the reaction conditions described above in Example 1(c), 1-amino-5-(6-ethoxycarbonylhexyl)hydantoin (1.08 g) and phenylglyoxal monohydrate (608 mg) in methanol (10 ml) yielded 5-(6-ethoxycarbonylhexyl)-1-phenacylideneamino) hydantoin, (Compound No. 13) m.p. 77-79°.

A solution of this ester (1.45 g) was hydrolysed in 0.5N-sodium hydroxide solution (22.0 ml) at room temperature for 1 hour. The resultant solution was washed with ether and acidified with dilute hydrochloric acid to yield 5-(6- carboxyhexyl)-1-(phenacylideneamino)hydantoin (Compound No. 14) as colourless prisms, m.p. 84-86°C, from ethyl acetate-light petroleum (b.p. 60-80°C).

Reduction of the above keto-acid proceeded as in Example 1(c), Method (i) to yield a mixture of the two diastereoisomers of (Compound No. 11 and Compound No.12) described separately in Method (i) above.

Example 4

Preparation of 5-(6-Carboxyhexyl)-1-(2-hydroxyheptylideneamino)hydantoin

Method (i)

A solution of 1-amino-5-(6-ethoxycarbonylhexyl)hydantoin (271 mg) and α-acetoxyheptaldehyde (Riehl and Fougerousse, Bull. Soc. Chim., 1968, 4083) in

methanol (4 ml) was refluxed for 2 hours. The solvent was evaporated and the residue was purified by chromatography (silica; chloroform - methanol (50:1)) to give 1-(2-acetoxyheptylideneamino)-5-(6-ethoxycarbonylhexyl)hydantoin as a colourless oily mixture of diastereoisomers showing spots at Rf 0.47 (Compound No. 15) and 0.51 (Compound No. 16) on thin layer chromatogaraphy (silica; chloroform - methanol (50:1)) N.m.r (CDCl$_3$): δ 2.21(5H, m, $CH_2CO_2$ and $COCH_3$), 4.11 (2H, qu, $CO_2$<u>$CH_2$</u>$CH_3$, 4.36 (1H, t, N-CH), 5.28 (1H, m, CH-0), 8.13 and 8.23 (1H, 2d, N=CH (two diastereoisomers)).

The above mixture of acetoxy-esters (213 mg) was hydrolysed with dilute sodium hydroxide solution (8.0 ml of 0.2N) at room temperature for 3 hours. The solution was washed with ether, then acidified with 2N-hydrochloric acid. The precipitated oil was extracted into ether, the extract was washed with water, dried (magnesium sulphate) and evaporated to give 5-(6-carboxyhexyl)-1-(2-hydroxyheptylideneamino)hydantoin (180 mg) as a mixture of diastereoisomers showing spots at Rf 0.41 (Compound No. 17) and 0.44 (Compound No. 18) on thin layer chromatography (silica; chloroform - methanol-acetic acid (90:5:5)). A solution of this mixture in ether, kept at 5$^{o}$C overnight, deposited crystals, m.p. 73.5$^{o}$C, of the pure diastereoisomer of Rf 0.41, (Compound No. 17) N.m.r. (CDCl$_3$): δ 2.35 (2H, t, $CH_2CO_2$), 4.37 (2H, m, N-CH and CH-O), 8.21 (1H, d, N=CH).

Method (ii)

A solution of 1-amino-5-(6-ethoxycarbonylhexyl)hydantoin (1.8 g) and pentylglyoxal (Royals and Robinson, J. Amer.Chem.Soc., 1956, 78, 4161) (1.1 g) in methanol (18 ml) was allowed to stand at room temperature for 40 hours. The solvent was evaporated, the residue was dissolved in ether and the solution was washed with 0.5N-hydrochloric acid, then with water, dried (magnesium sulphate) and evaporated. The residue was purified by chromatography (silica; chloroform) to give 5-(6-ethoxycarbonylhexyl)-1-(hexanoylmethyleneamino)hydantoin (Compound No.19), crystallising from ether-light petroleum (b.p. 40-60$^{o}$C) as colourless needles, m.p. 52-55$^{o}$C.

A solution of this ester (1.0 g) in 0.5N-sodium hydroxide solution (12.8 ml) was left at room temperature for 1 hours. The solution was washed with ether, acidified with dilute hydrochloric acid and the precipitated oil was extracted into ether. The washed and dried ether solution was evaporated to leave a viscous yellow oil

which was freed from minor impurities by chromatography (silica; chloroform-methanol (50:1)). The pure 5-(6-carboxyhexyl)-1-(hexanoylmethyleneamino)hydantoin (Compound No. 20) formed a hemihydrate, m.p. 84-85°C.

Reduction of the above keto-acid proceeded as in Example 1(c), Method (i) to yield the mixture of diastereoisomers of Compounds Nos. 17 and 18 as produced in Method (i) above.

Example 5

Preparation of 5-(6-carboxyhexyl)-1-(2-hydroxy-3-phenylpropylideneamino) hydantoin

A solution of 1-amino-5-(6-ethoxycarbonylhexyl) hydantoin (1.9g) and 2-acetoxy-3-phenylpropanal (1.35g) in methanol (28ml) was refluxed for 5 hours. The solvent was evaporated and the residue was purified by chromatography (silica; chloroform-methanol (50:1)) to give 1-(2-acetoxy-3-phenylpropylideneamino)-5-(6-ethoxycarbonylhexyl)hydantoin as a colourless oil. Thin-layer chromatography (silica; chloroform-methanol (50:1)) showed the presence of two diastereoisomers, Rf 0.54 (Compound No 21) and Rf 0.60 (Compound No 22). Separation of the isomers by high performance liquid chromatography (silica; chloroform-dichloromethane (5:1)) gave the more polar diastereoisomer. n.m.r. ($CDCl_3$) :δ 2.06 (3H,s,$COCH_3$), 2.28 (2H,t,$CH_2CO_2$), 3.12 (2H,d, $CH_2Ph$), 4.13 (2H, qu, $CO_2\underline{C}H_2CH_3$), 4.32 (1H,t,N-CH), 5.56 (1H,m,CH-O), 7.28 (5H,s,Ph), 8.23 (1H,d, N=CH) and the less polar diastereoisomer 2.05 (3H,s, $COCH_3$), 2.28 (2H,t,$CH_2CO_2$) 3.12(2H,d,$CH_2Ph$), 4.12 (2H,qu, $CO_2\underline{CH_2}CH_3$), 4.32 (1H,t,N-CH), 5.58 (1H,m, CH-O), 7.27 (5H,s,Ph), 8.09(1H,d,N=CH).

A mixture of the above diastereoisomeric acetoxy-esters was hydrolysed as described in Example 3 (Method (i)) to give a mixture of the diastereoisomers of 5-(6-carboxyhexyl)-1-(2-hydroxy-3-phenylpropylideneamino)hydantoin (Compounds Nos. 23 and 24). N.m.r. ($(CD_3)_2SO$, 80°C): δ 2.20 (2H,t,$\underline{CH_2}CO_2$), 4.40 (2H,m, N-CH and CH-O), 7.25 (5H,s,Ph), 8.64 and 8.80 (1H, 2d N=CH(two diastereoisomers)).

The pure more polar acetoxy-ester (Compound No 22) was similarly hydrolysed to give a single diastereoisomer of 5-(6-carboxyhexyl)-1-(2-hydroxy-3-phenyl propylideneamino)hydantoin crystallising from ethyl acetate in colourless needles, m.p. 116-118°C (Compound No. 23).

RMW/KMS/16.01.84

Example 6

Preparation of 5-(6-carboxyhexyl)-1-(2-cyclohexyl-2-hydroxyethylideneamino)-3-methyl hydantoin

A solution of 5-(6-carboxyhexyl)-1-(2-cyclohexyl-2-hydroxyethylideneamino)hydantoin (mixture of diastereoisomers; Compound No.3 and Compound No.6, Example 1) (350mg) in methanol (4.2ml) was treated with 2N-sodium hydroxide solution (1.05ml) and then methyl iodide (0.18ml) and allowed to stand at 20°C for 44 hours. The solvent was evaporated, water was added and the solution was washed with ether. The aqueous solution was acidfied and the precipitated oil was extracted into ether. The washed and dried ether solution was evaporated to leave an oil which was freed from traces of impurity by chromatography on a column of silica in chloroform-methanol (30:1). The 5-(6-carboxyhexyl)-1-(2-cyclohexyl-2-hydroxyethylideneamino)-3-methylhydantoin so obtained showed two spots on thin layer chromatography (silica; ether-light petroleum (b.p.60-80°C)-methanol-acetic acid (55:35:5:5), Rf 0.39 (Compound No. 25) and Rf 0.43 (Compound No 26) corresponding to the two diastereoisomers present. N.m.r. ($CDCl_3$): $\delta$ 2.35 (2H,t,$CH_2CO_2$), 3.05(3H,s, N-$CH_3$), 4.19 (1H,t, CH-O), 4.35 (1H,t,N-CH), 8.22 and 8.27 (1H, 2d, N=CH).

Example 7

Preparation of 5-(6-carboxyhexyl)-1-(2-cyclohexylethylamino)hydantoin

A solution of 1-(2-cyclohexylethylideneamino)-5-(6-ethoxycarbonylhexyl)hydantoin (Compound No 7, Example 2) (190 mg) and sodium cyanoborohydride (35 mg) in methanol (3.8 ml) containing acetic acid (0.2 ml) was allowed to stand at room temperature for 5 days. Water and ether were added, and the ether layer was washed with sodium bicarbonate solution, then with water, dried (magnesium sulphate) and evaporated to leave 5-(6-ethoxycarbonylhexyl)-1-(2-cyclohexyl-ethylamino)hydantoin (Compound No. 27) as a colourless oil.

This ester (185 mg) was hydrolysed in 0.2N-sodium hydroxide solution (5.5 ml) at room temperature for 3 hours. The product, isolated by neutralisation of the solution and ether extraction, was 5-(6-carboxyhexyl)-1-(2-cyclohexylethylamino)hydantoin (Compound No. 28) crystallising from ether-light petroleum (b.p. 60-80°C) as small colourless needles, m.p. 89-91°C.

RMW/KMS/16.01.84

## Example 8

## Preparation of 5-(6-carboxyhexyl)-1-(2-cyclohexyl-2-hydroxyethylamino)-hydantoin

A solution of 5-(-6-carboxyhexyl)-1-(2-cyclohexyl-2-hydroxyethylideneamino)-hydantoin (Compound No 3, Example 1) (96 mg) and sodium cyanoborohydride (1.6mg) in methanol (1.5ml) with acetic acid (0.1ml) was allowed to stand at 20°C for 4 days. The product was isolated by ether extraction, and recrystallised from ethyl acetate-light petroleum (b.p. 60-80°C) to give small prisms, m.p. 103-105°C, of one diastereoisomer of 5-(6-carboxyhexyl)-1-(2-cyclohexyl-2-hydroxyethylamino)hydantoin (Compound No.29).

In similar fashion, Compound No. 6, Example 1, was reduced to give the second diastereoisomer of 5-(6-carboxyhexyl)-1-(2-cyclohexyl-2-hydroxyethylamino) hydantoin (Compound No. 30) as a colourless viscous oil (n.m.r.($(CD_3)_2SO$): $\delta$2.18 (2H,t,$CH_2CO_2$), 2.82 (2H,m,N-$CH_2$), 3.25(1H,m, CH-O), 4.05(1H,t,N-CH).

## Example 9

## Preparation of 5-(6-carboxyhexyl)-1-(2-hydroxy-2-phenylethylamino) hydantoin

### Method (i)

5-(6-Ethoxycarbonylhexyl)-1-(2-acetoxy-2-phenylethylideneamino)hydantoin (mixture of Compound No.9 and Compound No.10, Example 3) was reduced with sodium cyanoborohydride as described in Example 8. The product was a colourless viscous oil, consisting of a mixture of the two diastereoisomers of 5-(6-ethoxycarbonylhexyl)-1-(2-acetoxy-2-phenylethylamino)hydantoin (Compound No. 31 and Compound No. 32). N.m.r. ($CDCl_3$): $\delta$ 2.1 (3H,s,$COCH_3$) 2.28 (2H,t,$CH_2CO_2$), 3.32(2H,m,N-$CH_2$), 3.95 (1H,t,N-CH), 4.14(2H,qu, $CO_2\underline{CH_2}CH_3$), 5.86 (1H,m,CH-O), 7.35(5H,s,Ph).

Hydrolysis of this acetoxy-ester with dilute sodium hydroxide solution as described in Example 7 gave as a viscous oil 5-(6-carboxyhexyl)-1-(2-hydroxy-2-phenylethylamino) hydantoin, a mixture of two diastereoisomers (Compound No. 33 and Compound No.34). N.m.r. ($(CD_3)_2SO$) : $\delta$ 2.16 (2H,t, $CH_2CO_2$), 2.8-3.2(2H,m,N-$CH_2$), 3.97(1H,m,N-CH), 4.57(1H,m,CH-O), 7.32 (5H,s,Ph).

RMW/KMS/16.01.84

Method (ii)

5-(6-Carboxyhexyl)-1-(2-hydroxy-2-phenylethylideneamino)hydantoin (Compound No. 11, Example 3) was reduced with sodium cyanoborohydride as described in Example 8. The product, a viscous oil, was one diastereoisomer of 5-(6-carboxyhexyl)-1-(2-hydroxy-2-phenylethylamino)hydantoin (Compound No. 33). N.m.r. ($(CD_3)_2SO$): $\delta$ 2.18 (2H, t, $CH_2CO_2$), 2.87 and 3.06 (each 1H, m, non-equivalent N-$CH_2$), 4.07 (1H, t, N-CH), 4.60 (1H, m, CH-O), 7.33 (5H, m, Ph).

Similarly, sodium cyanoborohydride reduction of the other diastereoisomer of 5-(6-carboxyhexyl)-1-(2-hydroxy-2-phenylethylideneamino)hydantoin (Compound No. 12, Example 3) gave the second diastereoisomer of 5-(6-carboxyhexyl)-1-(2-hydroxy-2-phenylethylamino)hydantoin (Compound No. 34), m.p. 112-114$^{o}$.

Example 10

Preparation of 5-(6-carboxyhexyl)-1-(2-hydroxyheptylamino) hydantoin

5-(6-Carboxyhexyl)-1-(2-hydroxyheptylideneamino)hydantoin (mixture of Compound No. 17 and Compound No. 18, Example 4) was reduced with sodium cyano-borohydride as described in Example 8. The product was a viscous oil consisting of a mixture of the two diastereoisomers of 5-(6-carboxyhexyl)-1-(2-hydroxyheptylamino)hydantoin (Compound No. 35 and Compound No. 36). N.m.r. ($(CD_3)_2SO$) : $\delta$ 2.17 (2H,t,$CH_2CO_2$), 2.71 and 2.81 (2H, 2m, N-$CH_2$), 3.45 (1H,br, CH-O), 4.05 and 4.08 (1H,2t, N-CH).

Similarly, sodium cyanoborohydride reduction of the diastereoisomer, m.p. 73.5$^{o}$C, of 5-(6-carboxyhexyl)-1-(2-hydroxyheptylideneamino)hydantoin (Compound No.17) gave a single diastereoisomer of 5-(6-carboxyhexyl)-1-(2-hydroxyheptylamino)hydantoin, m.p. 78-81$^{o}$C (Compound No. 35). N.m.r. ($(CD_3)_2SO$) : $\delta$ 2.18(2H,t,$CH_2CO_2$), 2.72 and 2.82 (2H, 2m, N-$CH_2$), 3.45 (1H,br, CH-O), 4.07(1H,t,N-CH).

Example 11

Preparation of 5-(6-carboxyhexyl-1-(2-cyclohexyl-2-hydroxyethylamino)-3-methyl-hydantoin.

5-(6-Carboxyhexyl)-1-(2-cyclohexyl-2-hydroxyethylideneamino)-3-methylhydantoin (mixture of diastereoisomers, Compound No. 25 and Compound No. 26, Example 6)

was reduced with sodium cyanoborohydride as described in Example 8. The product was purified by chromatography (silica; chloroform-methanol (50:1)) to give a colourless viscous oil, consisting of a mixture of the diastereoisomers of 5-(6-carboxyhexyl-1-(2-cyclohexyl-2-hydroxyethylamino)-3-methylhydantoin (Compound No. 37 and Compound No 38). N.m.r. ($CDCl_3$) : δ 2.33 (2H,t,$CH_2CO_2$), 2.6-3.0 (2H,m,N-$CH_2$), 3.05 (3H, s, N-$CH_3$), 3.40 (1H, br, CH-O), 4.02 (1H,t,N-CH).

Example 12

Preparation of 5-(6-carboxyhexyl)-1-(2-hydroxy-3-phenylpropylamino)hydantoin

5-(6-Ethoxycarbonylhexyl)-1-(2-acetoxy-3-phenylpropylidenamino)hydantoin (mixture of Compound No.21 and Compound No.22, Example 5) was reduced with sodium cyanoborohydride as described in Example 8. The product was a viscous oil, consisting of a mixture of the two diastereoisomers of 5-(6-ethoxycarbonylhexyl)-1-(2-acetoxy-3-phenylpropylamino)hydantoin (Compound No. 39 and Compound No.40)N.m.r. ($CDCl_3$): δ 2.05 (3H, s, $COCH_3$), 2.28 (2H, t, $CH_2$ $CO_2$), 2.92 (2H, d, $C\underline{H}_2$ Ph), 3.10 (2H, d, N-$CH_2$), 4.11 (2H, q, $CO_2$ $C\underline{H}_2$ $CH_3$), 4.35 (1H, t, N-CH), 5.15 (1H, m, CH-O), 7.25 (5H, s, Ph).

The above mixture of esters (140 mg) was hydrolysed with dilute sodium hydroxide solution (5 ml of 0.2N) at room temperature for 3 hours. The mixture was neutralised by additon of 2N-hydrochloric acid (0.5 ml) and the precipitated oil was extracted with ether. The washed and dried extract was evaporated to give a mixture of the diastereoisomers of 5-(6-carboxyhexyl)-1-(2-hydroxy-3-phenylpropylamino)hydantoin (Compound No. 41 and Compound No. 42) as a viscous oil .N.m.r. ($(CD_3)_2SO$): δ 2.18 (2H, t, $CH_2CO_2$), ca 2.6-2.85 (4H, m, Ph$C\underline{H}_2$ + N-$CH_2$), 3.17 (1H, m, CH-O), 4.04 (½H, t, one isomer N-CH), 4.09 (½H, t, one isomer N-CH), 7.23 (5H, m, Ph).

A solution of the above pair of diastereoisomers in a mixture of ethyl acetate and light petroleum (b.p. 60-80$^0$), kept in the refrigerator overnight, deposited crystals of one of the pure diastereoisomers, m.p. 93-95$^0$ (Compound No. 41), N.m.r. ($(CD_3)_2SO$): δ 2.17 (2H, t, $CH_2CO_2$), ca 2.6 - 2.85 (4H, m, Ph$C\underline{H}_2$ + N-$CH_2$), 3.17(1H, m, CH-O), 4.09 (1H, t, N-CH), 7.22 (5H, m, Ph).

Example 13

Preparation of 5-(6-carboxyhexyl)-1-(2-methoxy-2-phenylethylideneamino)hydantoin

1-Amino-5-(6-ethoxycarbonylhexyl)hydantoin and α-methoxyphenylacetaldehyde (Dornow and Muller, Chem. Ber., 1960, 93, 320) reacted together under the conditions of Example 3, Method (i) to give 5-(6-ethoxycarbonylhexyl)-1-(2-methoxy-2-phenylethylideneamino)hydantoin as an oily mixture of diastereoisomers showing spots at Rf 0.52 (Compound No. 43) and 0.56 (Compound No. 44) on thin-layer chromatography (silica; chloroform-methanol (19:1)) N.m.r. ($CDCl_3$): δ2.24(2H, t, $CH_2CO_2$), 3.40 (3H, s, $OCH_3$), 4.12 (2H, qu, $CO_2\underline{CH_2}CH_3$), 4.39 (1H, m, N-CH), 4.83 (1H, d, CH-O), 7.36 (5H, s, Ph), 7.83 and 7.87 (1H, 2d N=CH (two diastereoisomers)).

Hydrolysis of the foregoing mixture of esters with aqueous sodium hydroxide as described in Example 2 gave a mixture of the diastereoisomers of 5-(6-carboxyhexyl)-1-(2-methoxy-2-phenylethylidineamino) hydantoin (Compound No 45 and Compound No. 46) as a viscous oil. N.m.r. ($CDCl_3$): 2.30 (2H, m, $CH_2CO_2$), 3.42 (3H, s, $OCH_3$), 4.40 (1H, m, N-CH), 4.86 (1H, d, CH-O), 7.36 (5H, s, Ph), 7.77 and 7.84 (1H, 2d, N=CH (two diastereoisomers)).

Example 14

Preparation of 5-(6-carboxyhexyl)-1-(2-methoxy-2-phenylethylamino)hydantoin

5-(6-Carboxyhexyl)-1-(2-methoxy-2-phenylethylideneamino)hydantoin (mixture of Compound No. 45 and Compound No. 46, Example 13) was reduced with sodium cyanoborohydride as described in Example 8. The product, a viscous oil, was a mixture of the diastereoisomers of 5-(6-carboxyhexyl)-1-(2-methoxy-2-phenylethylamino) hydantoin (Compound No. 47 and Compound No. 48). N.m.r. ($CDCl_3$): δ 2.33 (2H, t, $CH_2CO_2$), 2.87 and 3.02 (2H, 2d, $NHCH_2$), 3.26 (3H, s, $OCH_3$), 4.01 (1H, t, N-CH), 4.30 and 4.39 (1H, 2d, CH-O), 7.30 (5H, s, Ph).

IN VITRO ACTIVITY

Inhibition of platelet aggregation

Human blood was freshly collected into siliconized (Siloclad; Clay Adams) plastic (Sterilin Ltd.) vessels containing trisodium citrate (3.15%; 0.1 volume with 0.9 volume blood) and centrifuged (200 g for 15 min) at room temperature. The platelet-rich plasma (PRP) was withdrawn into plastic containers and kept at room temperature. Inhibition of platelet aggregation was determined in a Born-type aggregometer by incubating aliquots (0.5 ml) of the PRP for 1 min at 37°C with or without the hydantoin prior to addition of sufficient adenosine diphosphate (ADP) or other aggregating agent to just cause a non-reversing control aggregation. Dose-inhibition curves were constructed for each compound and the $ID_{50}$ (dose causing 50% inhibition) was calculated as the dose required to reduce the aggregation to 50% of its control amplitude. Results show potency following the 1 minute incubation in PRP and the increase in potency following 5-10 minutes incubation in PRP. For comparison, also given are the results upon administration of 5-(6-carboxyhexyl)-1-(3-cyclohexyl-3-hydroxypropyl)hydantoin (Compound X) which is disclosed in UK Specification 1,595,694.

In this and subsequent biological examples, where a compound number refers to a single diastereoisomer, a mixture of diastereoisomers is indicated by two compound numbers separated by /.

| Compound No. | $IC_{50}$ ($ngml^{-1}$) | Potency relative to $PGI_2$ | Increase on Incubation (min) 5 | 10 |
|---|---|---|---|---|
| 3 | 16 | 0.02 | x7 | x20 |
| 6 | 2 | 0.30 | x5 | x20 |
| 8 | 24 | 0.05 | - | - |
| 11 | 1 | 0.53 | x8 | - |
| 17 | 131 | 0.01 | x2 | - |
| 17/18 | 8 | 0.12 | x2 | - |

| Compound No. | $IC_{50}$ ($ngml^{-1}$) | Potency relative to $PGI_2$ | Increase on Incubation (min) 5 | 10 |
|---|---|---|---|---|
| 25/26 | 13 | 0.06 | x6 | - |
| 29 | 6 | 0.16 | x7 | - |
| 30 | 0.9 | 0.64 | x6 | - |
| 33 | 5.3 | 0.07 | x2 | - |
| 34 | 175 | 0.003 | - | - |
| 33/34 | 6 | 0.17 | x2 | - |
| 35 | 12.7 | 0.05 | x1.5 | - |
| 35/36 | 1.27 | 0.49 | x1.5 | - |
| 37/38 | 3 | 0.18 | x7 | - |
| X | 1.5 | 0.21 | x2 | x3 |

## EX VIVO ACTIVITY

### Determination of rabbit platelet aggregation and concurrent change in systemic arterial blood presure

Male rabbits (2-2.5 kg body weight) were anaesthetized with sodium pentobarbitone (30 mg/kg, i.v. followed by maintenance doses of 3mg/kg) and systemic arterial blood pressure (BP) was recorded from a cannula filled with

heparinized saline (5 units/ml) in a carotid artery; no heparin was administered to the animal. Hydantoins were administered via a cannula in a femoral vein. Blood samples (1.5 ml) were slowly collected into a plastic syringe containing tri-sodium citrate (3.18%, 0.15 ml) from a cannula inserted into a femoral artery, shaken gently

and transferred to an Eppendorf plastic tube and spun in a modified Eppendorf centrifuge for 2 sec (Model 5412, maximum centrifugal force 10,000 x g). The PRP was collected and a 0.35 ml aliquot was transferred to the aggregometer and incubated at 37°C for 1 minute prior to addition of sufficient ADP (20 μM) to produce near-maximal aggregation. The time-interval between removal of blood samples and the transference of the PRP to the aggregometer was less than 1.5 min.

Blood samples were removed at 10 min. intervals once the BP of the rabbit had reached steady resting levels, 10-20 min. following the preparative surgical procedures. After three control blood samples had been prepared and the control aggregation of the platelets measured, the hydantoin was infused intravenously for 15 min from a Braun slow-infusion apparatus. Blood samples were collected 10 min after the start of the infusion. Two further 10 min. samples post-infusion were collected prior to commencing further infusions.

The following results list values of $ED_{50}$ and change in blood pressure. For comparison, also given are the results obtained upon administration of $PGI_2$ and Compound X.

| Compound | $ED_{50}$ ($\mu g\ kg^{-1} min^{-1}$) | $\Delta$BP (mmHg) |
|---|---|---|
| 3 | 2.0 | 0 |
| 6 | 0.15 | 0 |
| X | 3.3 | -5 |
| $PGI_2$ | 0.2 | -20 |

Thus, in this test, the $ED_{50}$ values for Compounds 3 and 6 were comparable to those for Compound X and $PGI_2$. However, at these doses, compounds 3 and 6 demonstrated no significant blood pressure depressant activity, in contrast to the two known agents.

RMW/KMS/16.01.84

IN VIVO ACTIVITY

(a) Cardiovascular actions in rats

Anaesthesia was induced in male Wistar rats (250-300 g body weight) with sodium pentobarbitone (30 mg $kg^{-1}$, i.v.) and supplemented (3 mg $kg^{-1}$) when required. Arterial pressure was recorded from a cannulated femoral artery and heart rate derived from the arterial pulse. Rectal temperature was maintained at 37°C by thermistor-controlled radiant heat. Each compound was injected into a femoral vein in a volume of 0.25 ml and flushed in with 0.2 ml of saline (0.9% w/v). Dose-response relationships for the fall in mean systemic arterial blood pressure (BP) with the hydantoins were constructed and compared to those obtained with $PGI_2$ and $PGE_1$ in the same animal, and a potency ratio calculated. An index of the duration of the hypotensive effect for each compound was obtained by measuring time from the peak response to when it had recovered to half its peak response ($t_{\frac{1}{2}}$ min). Figures for Compound X and $PGI_2$ are listed for comparison.

| Compound | Fall in BP Relative Potency | | Duration $t_{\frac{1}{2}}$ (mins) |
|---|---|---|---|
| | $PGI_2$ | $PGE_1$ | |
| 3 | 0.027 | 0.324 | 11.0 |
| 6 | 0.041 | 0.5 | 17.0 |
| 8 | 0.01 | 0.11 | 1.0 |
| 11 | 0.1 | 1.1 | 16.0 |
| 17 | 0.01 | 0.12 | 3.5 |
| 17/18 | 0.04 | 0.48 | 10-19 |
| 25/26 | 0.09 | 1.08 | 9 |
| 29 | 0.02 | 0.24 | 37 |
| 30 | 0.02 | 0.24 | 28 |
| 33/34 | 0.02 | 0.24 | 6 |
| 35 | 0.01 | - | 1.3 |
| 35/36 | 0.06 | - | 0.1 |
| 37/38 | 0.03 | 0.36 | 17 |
| X | 0.06 | 0.72 | 4.0 |
| $PGI_2$ | 1 | 11.0 | 0.3 |

RMW/KMS/16.01.84

(b) Cardiovascular studies in the dog

Dogs (10-15 kg body weight) were anaesthetized with chloralose (80 mg/kg i.v.) and maintained with subcutaneous injections of pentobarbitone (3 mg/kg). Drugs were administered via a femoral vein by bolus injection and B.P. was recorded from the right femoral artery. Results give vasodepressor potency and $t_{\frac{1}{2}}$ duration. Figures for Compound X are provided for comparison.

| Compound | Dose (ug $kg^{-1}$) | $\Delta$ BP (mm Hg) | $t_{\frac{1}{2}}$ (min) |
|---|---|---|---|
| X | 1.25 | -45 | 2.8 |
| | 2.5 | -56 | 6.2 |
| 6 | 0.625 | -54 | 60 |
| | 1.25 | -65 | >60 |
| 29 | 0.625 | -26 | >30 |
| | 1.25 | -51 | >60 |

(c) Inhibition of gastric lesions in rats

The inhibition of acid-ethanol induced gastric lesions in rats (cytoprotection) was measured for various doses of orally administered hydantoin. Figures for compound X are given for comparison.

| Compound | Dose µg $kg^{-1}$ | % inhibition |
|---|---|---|
| 4/5 | 50 | 90 ± 6 |
| 6 | 10 | 31 ± 20 |
| | 25 | 50 ± 21 |
| | 50 | 69 ± 8 |
| 8 | 25 | 71 ± 6 |
| | 50 | 76 ± 11 |
| X | 5 | 46 ± 26 |
| | 10 | 80 ± 9 |
| | 25 | 89 ± 6 |
| 23/24 | 10 | 55 ± 15 |
| 30 | 25 | 65 ± 17 |
| 37/38 | 10 | 71 ± 7 |

The above compounds also exhibited oral anti-ulcer activity against 3-hour indomethacin (20mg/Kg)-induced gastric erosions with comparable potency to that listed above.

## PHARMACEUTICAL FORMULATIONS

The following Examples illustrate pharmaceutical compositions according to the present invention. In Examples A-D, the "Compound" is a hydantoin of formula (I) (including physiologically acceptable salts and esters) which exists in the solid phase at normal room temperature. In Examples E and F, the "Compound" is a hydantoin of formula (I) (including physiologically acceptable salts and esters) which exists in either the solid or liquid phase at normal room temperature. In examples G-J the "Compound" is a hydantoin of formula (I) including physiologically acceptable salts and esters which exist in the liquid phase (eg as oils) at normal room temperature. Such liquid compounds may be any of compounds 4-6, 9, 10, 15, 16, 21, 22, 25-27, 30-36, 37, 38 or mixtures thereof which are liquids at said room temperature.

Example A

| Tablet | In one tablet |
|---|---|
| Compound | 5.0 mg |
| Lactose B.P. | 82.0 mg |
| Starch B.P. | 10.0 mg |
| Povidone B.P.C. | 2.0 mg |
| Magnesium Stearate | 1.0 mg |

Mix together the compound, lactose and starch. Granulate the powders using a solution of the povidone in Purified Water. Dry the granules, add the Magnesium Stearate and compress to produce tablets, 100 mg per tablet.

Example B

| Capsule | In one capsule |
|---|---|
| Compound | 10 mg |
| Lactose | 79 mg |
| Starch | 10 mg |
| Magnesium Stearate | 1 mg |

Mix the powders in a powder blender, fill into hard gel capsules, 100 mg per capsule.

Example C

1 µg/ml Injection

| | | |
|---|---|---|
| Compound | 100 µg | |
| Water for Injections | to....... | 100 ml |

Dissolve the compound in the Water for Injections. Sterilise the solution by filtration through a membrane filter, 0.22 µm pore size, collecting the filtrate in a sterile receiver. Under aseptic conditions, fill the solution into sterile glass ampoules, 1 ml per ampoule. Seal by fusion of the glass.

Example D

10 µg/ml Injection

| | |
|---|---|
| Compound | 1 mg |
| Ethyl Alcohol | 10 ml |
| Propylene Glycol | 30 ml |
| Water for Injections | to.......100 ml |

Dissolve the compound in the ethyl alcohol, add the propylene glycol and dilute to volume with Water for Injections.

Sterilise the solution by filtration through a membrane filter, 0.22 µm pore size, collecting the filtrate in a sterile vessel. Under aseptic conditions, fill the solution into sterile glass vials, 10 ml per vial. Close with a sterile rubber plug and secure with an aluminium collar.

Example E

100 µg Single dose injection (freeze-dried)

| | |
|---|---|
| Compound | 10.0 mg |
| Mannitol | 2.5 g |
| N/10 Sodium Hydroxide Solution | qs to pH 10.0 |
| Water for Injections | to........100.0 ml |

RMW/KMS/16.01.84

Suspend the compound in approximately 20 ml Water (or in the case of Compound No.11, mix the compound and the water to form a crude emulsion). Add sufficient Sodium Hydroxide Solution to produce pH 10 and stir to dissolve the compound. Add and dissolve the Mannitol and dilute to volume with Water for Injections.

Sterilise the solution by passage through a membrane filter, 0.22 µm pore size and distribute aseptically into sterile vials, 1 ml per vial. Freeze-dry the solutions and seal the containers under aseptic conditions with rubber closures. Each vial contains 100 ug compound as its freeze-dried sodium salt.

Example F

Suppository

| | |
|---|---|
| Compound | 3 mg |
| Massa Esterinum C | to........2 mg |

Melt the suppository base at around 40$^{\circ}$C. Gradually incorporate the compound in fine powder and mix until homogenous. Pour into suitable moulds and allow to set.

Massa Esterinum C is a commercially available suppository base consisting of a mixture of mono-, di- and tri-glycerides of saturated vegatable fatty acids. It is marketed by Henkel International, Dusseldorf.

Example G

Tablet

| | In one Tablet |
|---|---|
| Compound | 0.5 mg |
| Micro-crystalline cellulose excipient | 50 mg |
| Lactose B.P. | 36.5 mg |
| Starch B.P. | 10.0 mg |
| Povidone B.P.C.(polyvinylpyrrolidone) | 2.0 mg |
| Magnesium Stearate | 1.0 mg |

Dissolve the Compound in a suitable solvent and spread over the micro-crystalline cellulose excipient. Dry and mix together with the lactose and starch. Granulate the powders using a solution of the povidone in purified water. Dry the granules, add the magnesium stearate and compress to produce tablets, 100 mg per tablet.

RMW/KMS/16.01.84

Example H

| Capsule | In one Capsule |
|---|---|
| Compound | 0.5 mg |
| Maize oil | 99.5 mg |

Dissolve the Compound in the maize oil and fill into soft gelatin capsules, 100 mg per capsule.

Example I

1 µg/ml Injection

| | |
|---|---|
| Compound | 100 µg |
| Polysorbate 80 B.P. | as 0.1% w/v of final volume |
| Water for Injections | to.......100 ml |

Mix the polysorbate 80 with the Water for Injections and then mix-in the Compound to form an emulsion. Sterilise the emulsion by filtration through a membrane filter, 0.22 µm pore size, collecting the filtrate in a sterile receiver. Under aseptic conditions, fill the solution into sterile glass ampoules, 1 ml per ampoule. Seal by fusion of the glass.

Example J

10 µg/ml Injection

| | |
|---|---|
| Compound | 1 mg |
| Ethyl Alcohol | 10 ml |
| Propylene Glycol | 30 ml |
| Polysorbate 80 B.P. | as 0.1% w/v of final volume |
| Water for Injections | to........100 ml |

Mix the polysorbate 80 with 20 ml of the Water for Injections then mix-in the Compound to form an emulsion. Add the ethyl alcohol and the propylene glycol and mix thouroughly. Dilute to volume with Water for Injections. Sterilise the mixture by filtration through a membrane filter, 0.22 µm pore size, collecting the filtrate in a sterile vessel. Under aseptic conditions, fill the solution into sterile glass vials, 10 ml per vial. Close with a sterile rubber plug and secure with an aluminium collar.

RMW/KMS/16.01.84

Claims:-

1) A hydantoin of formula (I)

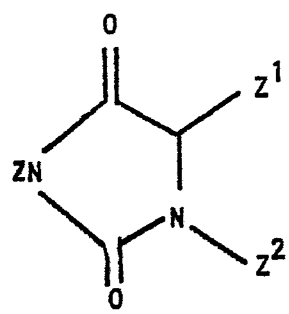

(I)

wherein Z is hydrogen or alkyl;

$Z^1$ represents a group of formula $-CH_2-X-X^1-X^2$ wherein, X is selected from $-(CH_2)_2-$, and cis and trans -CH=CH-, $X^1$ is a covalent bond or a straight or branched alkylene chain having 1 to 6 carbon atoms optionally having one of its methylene groups replaced by oxa (-O-) or thia (-S-) provided that at least one carbon atom separates the oxa or thia group from a carboxyl or -CH=CH- group, and $X^2$ is a carboxyl group;

and $Z^2$ represents a group selected from $-NH-CH_2-R$ and -N=CH-R wherein, R is a group selected from -CO-Y and $-\underset{Y^1}{\underset{|}{CH}}-Y$, Y being a group selected from $C_{3-8}$ alkyl, $C_{3-8}$ alkenyl, phenyl-$C_{1-4}$ alkyl and phenyl (wherein the phenyl group in both cases is optionally substituted by one or more groups independently selected from alkyl, alkoxy, nitro, halo and trihalomethyl), cycloalkyl of 4 to 8 carbon atoms, and 5- or 6- membered heterocyclic radicals containing at least one heteroatom selected from oxygen, sulphur and nitrogen; and
$Y^1$ being a group selected from hydrogen, hydroxy, alkoxy and acyloxy; and esters, amides, salts and solvates thereof.

RMW/KMS/17.01.84

2) A compound which is a bioprecursor of a hydantoin as claimed in claim 1.

3) A hydantoin as claimed in claim 1 wherein $Z^2$ is a group $-NH-CH_2-R$ as defined.

4) A hydantoin as claimed in claim 1 wherein $Z^2$ is a group $-NH=CH-R$ as defined.

5) A hydantoin as claimed in claim 1 wherein Z is hydrogen, $Z^1$ is $-CH_2-X-X^1-X^2$ wherein X is $-(CH_2)_2-$, $X^1$ is an alkylene chain of 2 to 4 carbon atoms and $X^2$ is carboxyl (or a corresponding alkyl ester) or a salt thereof;

and $Z^2$ is a group selected from $-NH-CH_2-R$ and $-N=CH-R$ wherein R is a group of formula $-\underset{Y^1}{\underset{|}{CH}}-Y$ in which $Y^1$ is hydroxy and Y is phenyl, alkyl of 3 to 8 carbon atoms or cycloalkyl of 4 to 8, particularly 6 carbon atoms.

6) A hydantoin as claimed in claim 1 or claim 5 selected from 5-(6-carboxy-hexyl)-1-(2-cyclohexyl-2-hydroxyethylamino)hydantoin, 5-(6-carboxy-hexyl)-1-(2-cyclohexyl-2-hydroxy-ethylideneamino)hydantoin, 5-(6-carboxy-hexyl)-1-(2-hydroxy-2-phenylethylideneamino)- hydantoin and esters, amides, salts and solvates thereof.

7) A single diastereoisomer of a hydantoin as claimed in claim 6, the diastereoisomer being a viscous oil at 21$^oC$.

8) A single diastereoisomer of a hydantoin as claimed in claim 6, the diastereoisomer being a crystalline solid at 21$^oC$.

9) A pharmaceutical formulation comprising a hydantoin as claimed in any of claims 1 and 3-6 or a pharmacologically acceptable ester, amide, salt or solvate thereof, and a pharmacologically acceptable carrier therefor.

10) A process for the preparation of a hydantoin of formula (I)

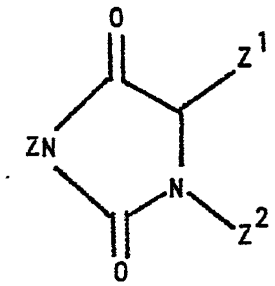

(I)

wherein Z is hydrogen or alkyl; $Z^1$ represents a group of formula $-CH_2-X-X^1-X^2$ wherein, X is selected from $-(CH_2)_2-$, and <u>cis</u> and <u>trans</u> $-CH{=}CH-$, $X^1$ is a covalent bond or a straight or branched alkylene chain having 1 to 6 carbon atoms optionally having one of its methylene groups replaced by oxa (-O-) or thia (-S-) provided that at least one carbon atom separates the oxa or thia group from a carboxyl or -CH=CH- group, and $X^2$ is a carboxyl group;

and $Z^2$ represents a group selected from $-NH-CH_2-R$ and -N=CH-R wherein, R is a group selected from -CO-Y and $-\underset{Y^1}{CH}-Y$, Y being a group selected from $C_{3-8}$

alkyl, $C_{3-8}$ alkenyl, phenyl-$C_{1-4}$ alkyl and phenyl (wherein the phenyl group in both cases is optionally substituted by one or more groups independently selected from alkyl, alkoxy, nitro, halo and trihalomethyl), cycloalkyl of 4 to 8 carbon atoms, and 5- or 6- membered heterocyclic radicals containing at least one heteroatom selected from oxygen, sulphur and nitrogen; and

$Y^1$ being a group selected from hydrogen, hydroxy, alkoxy and acyloxy; and esters, amides, salts and solvates thereof, which comprises:-

(a) for the preparation of hydantoins where $Z^2$ represents -N=CH-R, wherein R is as defined above, reacting of a compound of formula (II)

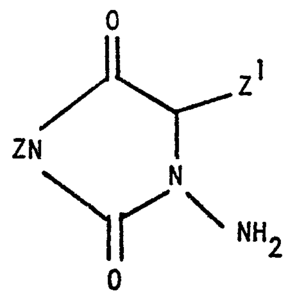

(II)

(wherein Z and $Z^1$ are as defined in formula (I)) with a compound of formula (III)

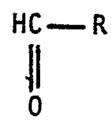

(III)

(wherein R is as defined in formula (I));

(b) for the preparation of hydantoins where $Z^2$ represents -N=CH-R, wherein R is as defined above, reacting an appropriate compound of formula (IV)

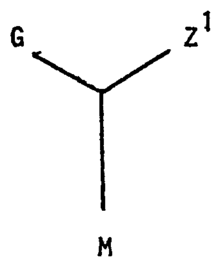

(IV)

(wherein $Z^1$ is as defined in formula (I), G is carboxy or a derivative thereof such as an amide derivative e.g. carbamoyl, or an ester thereof, particularly an alkyl ester or G may be cyano and M is a leaving group such as halo, preferably bromo) with a compound of formula (V)

ZHNCONHN=CHR (V)

(wherein R and Z are as defined in formula (I));

(c) for the preparation of hydantoins where $Z^2$ represents-N=CH-R, wherein R is as defined in formula (I), cyclising a compound of formula (VI)

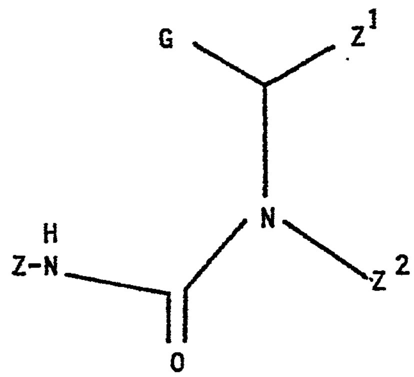

(VI)

wherein Z, $Z^1$ and $Z^2$ are as defined in formula (I) and G is as defined in formula (IV);

(d) for the preparation of hydantoins where $Z^2$ represents -N=CH-R, wherein R and Z are as defined in formula (I), treating a compound of formula (VII)

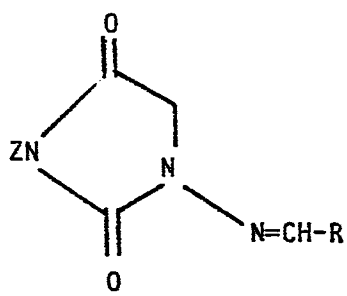

(VII)

(wherein R is as defined in formula (I)) with methylmagnesium carbonate followed by reaction with a compound of formula (VIII)

$M-Z^1$ (VIII)

(wherein $Z^1$ is as defined in formula (I) and M is defined in formula (IV)).

11) A process as claimed in claim 10 comprising subsequently effecting one or more of the following optional conversions in any desired order:-

i) where a hydantoin of formula (I) is formed and is an ester, converting said compound into a corresponding acid, or to a salt, solvate or amide thereof;

ii) where a hydantoin of formula (I) is formed and is an acid, converting said hydantoin into a corresponding ester, or to a salt, solvate or amide thereof;

iii) where a hydantoin of formula (I) is formed wherein Z is hydrogen, converting said hydantoin into a corresponding hydantoin wherein Z is alkyl;

iv) where a hydantoin of formula (I) is formed wherein R is $-\underset{\displaystyle Y^1}{\underset{|}{C}H}-Y$, wherein

$Y^1$ is an acyloxy group and Y is as defined above, converting said hydantoin into a corresponding hydantoin of formula (I) wherein $Y^1$ is a hydroxy group;

(v) where a hydantoin of formula (I) is formed wherein R is a group -CO-Y, converting the said hydantoin into a corresponding hydantoin of formula (I) wherein R is a group -CH(OH)-Y; or

(vi) converting the hydantoin of formula (I) to the corresponding hydantoin where $Z^2$ represents $-NH-CH_2-R$, wherein R is as defined above.

RMW/KMS/17.01.84

12) A compound of formula (II)

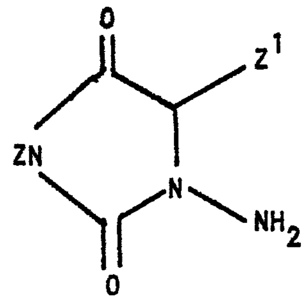

(II)

wherein Z is hydrogen or alkyl; and

$Z^1$ represents a group of formula $-CH_2-X-X^1-X^2$ wherein, X is selected from $-(CH_2)_2-$, and _cis_ and _trans_ -CH=CH-, $X^1$ is a covalent bond or a straight or branched alkylene chain having 1 to 6 carbon atoms optionally having one of its methylene groups replaced by oxa (-O-) or thia (-S-) provided that at least one carbon atom separates the oxa or thia group from a carboxyl or -CH=CH- group, and $X^2$ is a carboxyl group.

13) A compound of formula (IX)

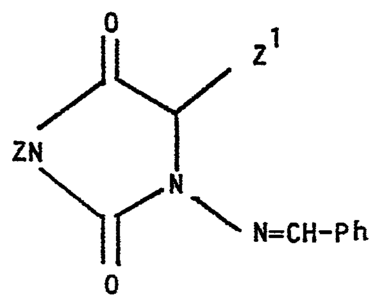

(IX)

wherein Z is hydrogen or alkyl;

$Z^1$ represents a group of formula $-CH_2-X-X^1-X^2$ wherein, X is selected from $-(CH_2)_2-$, and _cis_ and _trans_ -CH=CH-, $X^1$ is a covalent bond or a straight or branched alkylene chain having 1 to 6 carbon atoms optionally having one of its methylene groups replaced by oxa (-O-) or thia (-S-) provided that at least one carbon atom separates the oxa or thia group from a carboxyl or -CH=CH- group, and $X^2$ is a carboxyl group; and

Ph represents phenyl.

14) A hydantoin of formula (I) as defined in claim 1, or a pharmacologically acceptable ester, amide, salt or solvate thereof, for use in a method of prophylaxis or treatment of the human or animal body by therapy.

15) A hydantoin of formula (I) as defined in claim 1, or a pharmacologically acceptable ester, amide, salt or solvate thereof for use in a method of inhibiting the aggregation of blood platelets.

16) A hydantoin of formula (I) as defined in claim 1, or a pharmacologically acceptable ester, amide, salt or solvate thereof for inducing vasodilation in a mammal (including man).

RMW/KMS/17.01.84

European Patent Office

# EUROPEAN SEARCH REPORT

0126849
Application number

EP 84 10 1685

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | US-A-3 894 007 (WHITE) --- | | C 07 D 233/80<br>A 61 K 31/415 |
| A | GB-A-1 071 470 (BROCADES-STHEEMAN) --- | | |
| D,A | GB-A-1 595 694 (WELLCOME FOUNDATION) --- | | |
| D,A | GB-A-1 595 695 (WELLCOME FOUNDATION) ----- | | |
| | | | **TECHNICAL FIELDS SEARCHED (Int. Cl. 3)** |
| | | | C 07 D 233/00<br>A 61 K 31/00 |

The present search report has been drawn up for all claims

| Place of search | Date of completion of the search | Examiner |
|---|---|---|
| THE HAGUE | 18-05-1984 | DE BUYSER I.A.F. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document